Europäisches Patentamt

(19) European Patent Office

Office européen des brevets

(11) Publication number: 0 317 279
A2

(12) **EUROPEAN PATENT APPLICATION**

(21) Application number: 88310802.9

(22) Date of filing: 16.11.88

(51) Int. Cl.⁴: **C 07 K 7/08**
C 07 K 5/08, C 07 K 5/10,
C 07 K 7/06, C 07 K 1/14,
C 07 K 3/20

(30) Priority: **17.11.87 US 121461**

(43) Date of publication of application:
**24.05.89 Bulletin 89/21**

(84) Designated Contracting States:
**AT BE CH DE ES FR GB GR IT LI LU NL SE**

(71) Applicant: **SCRIPPS CLINIC AND RESEARCH FOUNDATION**
**10666 North Torrey Pines Road**
**La Jolla California 92037-1093 (US)**

(72) Inventor: **Zimmerman, Theodore S.**
**544 Bonair Street**
**La Jolla California 92037 (US)**

**Foster, Paul A.**
**8507 Capricon Way, Apt. 70**
**San Diego California 92126 (US)**

**Houghten, Richard A.**
**558 Ford Avenue**
**Solona Beach California 92075 (US)**

**Fulcher, Carol A.**
**7418 Fay Avenue**
**La Jolla California 92037 (US)**

(74) Representative: **Jump, Timothy John Simon et al**
**VENNER, SHIPLEY & CO. 368 City Road**
**London EC1V 2QA (GB)**

(54) **Peptides for use in the purification of factor VIII.**

(57) Peptides based on the amino acid sequence of Factor VIII which can be used for the purification of Factor VIII and methods for the preparation of these peptides are disclosed. Methods for the purification of Factor VIII using these peptides are also disclosed.

EP 0 317 279 A2

Description

## PEPTIDES FOR USE IN THE PURIFICATION OF FACTOR VIII

This invention relates to peptides based on the amino acid sequence of Factor VIII which can be used for the purification of Factor VIII.

The blood of individuals with hemophilia is defective with the result that it does not clot properly. The predominant type of hemophilia results from a defect in Factor VIII, Factor VIII being one of a series of proteins involved in the blood coagulation process. Exogenous Factor VIII can be administered to hemophiliacs with defective Factor VIII in order to correct their clotting defect.

Various methods have been developed for the purification of Factor VIII. However, there is a need for improved Factor VIII purification methods which result in higher yields of Factor VIII. The present invention accomplishes this by using peptides based on the amino acid sequence of Factor VIII for the purification of Factor VIII. This allows Factor VIII to be purified using milder conditions, resulting in an improved yield of Factor VIII.

The present invention relates to peptides based on the amino acid sequence of Factor VIII which can be used for the purification of Factor VIII. These peptides can be used to raise monoclonal or other antibodies for Factor VIII purification. These peptides can also be used to elute Factor VIII from monoclonal or other antibodies for the purification of Factor VIII.

Particularly preferred is a peptide of the following sequence:

TDSEMDVVRFDDDNS

whose amino acid sequence is that of amino acid residues 351-365 of the Factor VIII sequence.

Additionally preferred is a polypeptide of the following sequence:

EEIDYDDTISVEMKK

whose amino acid sequence is that of amino acid residues 1660-1674 of the Factor VIII sequence.

The invention further relates to a peptide comprising a sequential subset of at least three amino acid residues of a peptide based on the amino acid sequence of Factor VIII which can be used for the purification of Factor VIII.

The invention also relates to improved methods for the preparation of purified Factor VIII using peptides based on the amino acid sequence of Factor VIII and any sequential subset of at least three amino acids of the peptides.

The invention further relates to methods of preparing by recombinant DNA or synthetic peptide methods, peptides based on the amino acid sequence of Factor VIII and any sequential subset of at least three amino acids of the peptides.

As indicated, the present invention encompasses peptides based on the amino acid sequence of Factor VIII which can be used for the purification of Factor VIII. These peptides can be used to raise monoclonal or other antibodies for Factor VIII purification. These peptides can also be used to elute Factor VIII from monoclonal or other antibodies for the purification of Factor VIII.

The following description provides details of the manner in which the embodiments of the present invention may be made and used. This description, while exemplary of the present invention, is not to be construed as specifically limiting the invention and such variations which would be within the purview of one skilled in this art are to be considered to fall within the scope of this invention.

Various peptides based on the amino acid sequence of Factor VIII and a sequential subset of at least three amino acid residues of the peptides are suitable for use in the present invention. For example, the peptides TDSEMDVVRFDDDNS and EEIDYDDTISVEMKK and a sequential subset of at least three amino acid residues of either of these peptides may be used.

Peptides based on the amino acid sequence of Factor VIII and a sequential subset of at least three amino acid residues of the peptide are synthesized as described by Houghton et al., Proc. Natl. Acad. Sci. 82:5135 (1985).

In the well known procedure for solid-phase synthesis of a peptide, the desired peptide is assembled starting from an insoluble support such as benzhydryl amine or chloromethylated resin (derived from cross-linked polystyrene, and available from chemical supply houses). The amino acid at the carboxy-terminal end of the desired peptide, carrying protecting groups on the alpha-amino nitrogen and on any other reactive sites, is attached to the resin from solution using known peptide coupling techniques. The protecting group on the alpha-amino group is removed (leaving other protecting groups, if any, intact), and the next amino acid of the desired sequence (carrying suitable protecting groups) is attached, and so on. When the desired peptide has been completely built up, it is cleaved from the resin support, all protecting groups are removed, and the peptide is recovered. Examples of suitable protecting groups are: alpha-tert-butyloxycarbonyl for the alpha-amino-group; benzyl, 4-methoxybenzyl, or 4-methylbenzyl for the thiol group of cysteine, the beta-carboxylic acid group of aspartic acid, the gamma-carboxylic acid group of glutamic acid and the hydroxyl groups of serine, threonine, and tyrosine; benzyloxycarbonyl or a 2-chloro- or 3,4-dimethoxy-derivative thereof for the ring nitrogens of histidine and tryptophan and the epsilon-amino group of lysine; p-nitrophenyl for the amide nitrogens of asparagine and glutamine; and nitro or tosyl for the guanidine group of arginine.

For purposes of this disclosure, accepted shorthand designations of the amino acids have been used. A complete listing is provided herein below:

One and three-letter Amino Acid Abbreviations

| A | Ala | Alanine |
|---|-----|---------|
| C | Cys | Cysteine |
| D | Asp | Aspartic Acid |
| E | Glu | Glutamic Acid |
| F | Phe | Phenylalanine |
| G | Gly | Glycine |
| H | His | Histidine |
| I | Ile | Isoleucine |
| K | Lys | Lysine |
| L | Leu | Leucine |
| M | Met | Methionine |
| N | Asn | Asparagine |
| P | Pro | Proline |
| Q | Gln | Glutamine |
| R | Arg | Arginine |
| S | Ser | Serine |
| T | Thr | Threonine |
| V | Val | Valine |
| W | Trp | Tryptophan |
| Y | Tyr | Tyrosine |
| B | Asx | Asp or Asn, not distinguished |
| Z | Glx | Glu or Gln, not distinguished |
| X | X | Undetermined or atypical amino acid |

The complete amino acid sequence of Factor VIII has been determined (see Vehar et al., Structure of Human Factor VIII, Nature 312: 337-342 (1984)). With such information, a nucleotide sequence can be inserted into an appropriate vector for the expression of peptides based on the amino acid sequence of Factor VIII and any sequential subset of at least three amino acids of the peptides. For a description of recombinant DNA techniques for cloning Factor VIII fragments, see Wood et al., Nature 312: 330-336 (1984) and Toole et al., Nature 312: 342-346 (1984).

Various monoclonal antibodies are suitable for use in the present invention. For example, monoclonal antibodies with the ability to bind Factor VIII may be used. Also, monoclonal antibodies raised against peptides based on the amino acid sequence of Factor VIII and any sequential subset of at least three amino acids of the peptides may be used.

The monoclonal antibody C5 (previously described in Fulcher et al., Human Factor VIII Procoagulant Protein: Monoclonal Antibodies Define Precursor-Product Relationships and Functional Epitopes, J. Clin. Invest., Vol. 76, pp. 117-124 (1985)) is a high affinity antibody which binds to Factor VIII and, in doing so, neutralizes Factor VIII activity.

To produce the hybridoma cell line producing monoclonal antibody C5, Balb/c mice were immunized by intraperitoneal injection of 1 ug of either purified Factor VIII:C or thrombin-degraded purified Factor VIII:C in complete Freund's adjuvant. 10- and 50-ug boosters in incomplete Freund's adjuvant were given at 1-wk intervals. The final 100-ug booster was given without adjuvant 3 days before fusion. At 4 wk, the mouse spleen cells were fused with P3X63 mouse plasmacytoma cells as described in Brown et al., J. Biol. Chem. 255:4980-4983 (1980). Cell culture and production of monoclonal antibody in mouse ascites fluid were essentially by Liu et al., J. Immunol. 124:2728-2736 (1980). The antibodies were selected using a solid-phase assay in Linbro-Titertek (Flow Laboratories, Inglewood, CA) plates and an enzyme-linked immunosorbent detection system as described in Engvall and Perlmann, Immunochemistry 8:871-874 (1971) using a peroxidase-antibody conjugate (Zymed Laboratories, Burlingame, CA). The plates were coated with 100 ng of either purified Factor VIII:C or purified thrombin-degraded Factor VIII:C per well. Clones were also screened against plates coated with 100 ng of human fibrinogen, plates coated with 100 ng of human fibronectin, and plates coated with 100 ng of human von Willebrand factor per well, each protein being a potential contaminant of the immunogen.

Clones which were positive only on the Factor VIII:C-coated plates were also tested for their ability to inhibit plasma Factor VIII:C activity, using a modified Kasper assay (see Kasper et al., Thromb. Diath. Haemorrh. 34:869-872 (1975)). Culture supernatants were incubated with an equal volume of normal pooled human plasma, which had been diluted 1:10 or 1:20 into assay buffer as described in Fulcher et al., Proc. Natl. Acad. Sci. 79:1648-1652 (1982) to which had been added 2% (vol/vol) of 0.5 M sodium citrate, pH 6.5. Incubation was for 2 h at 37°C, after which samples were assayed for Factor VIII:C procoagulant activity. Culture supernatants from clone C5 inhibited 75% of the residual plasma Factor VIII:C activity, as compared with control (non-anti-Factor VIII:C supernatant) incubations.

Culture supernatants were tested for immunoglobulin class and subclass by double diffusion in agarose gels using commercially available antisera (Miles Laboratories, Inc., Elkhart, IN). Protein A-Sepharose chromatography (Pharmacia Fine Chemicals, Piscataway, NJ) of mouse ascites fluid was as described by Ey et al., Immochemistry 15:479-436 (1978). Purified antibodies were concentrated in a pressure ultrafiltration stirred cell (Amicon Corp., Danvers, MA) using a YM-10 membrane, aliquoted, and stored frozen at -70°C. Protein A-Sepharose purified monoclonal antibodies were assayed for their ability to inhibit plasma Factor VIII:C procoagulant activity using the modified Kasper assay (see Kasper et al., Thromb. Diath. Haemorrh. 34:869-872 (1975)).

The peptide EEIDYDDTISVEMKK was also used to raise monoclonal antibodies in mice.

Immunogen for the preparation of monoclonal antibodies to the peptide EEIDYDDTISVEMKK was prepared by coupling the polypeptide fragment to a carrier protein, keyhole limpet hemocyanin (KLH), using glutaraldehyde. Equal weights of the polypeptide and carrier protein were dissolved in phosphate buffered saline (PBS; 1.6 g monobasic sodium phosphate, 8.4 g dibasic sodium phosphate, 61.4 g sodium chloride, distilled water to 7 liters, pH 7.2) to a final concentration of 2 mg of carrier protein per ml of solution. Fresh glutaraldehyde working solution was prepared by adding 200 ml of a stock solution of 25% glutaraldehyde to 13 ml of PBS. 124 ul of fresh glutaraldehyde working solution was then added per 1.0 ml of carrier peptide solution. The resulting solution was stirred overnight at room temperature, dialyzed for at least 6 hours against distilled water and lyophilized.

Balb/c mice were immunized by intraperitoneal injection with 100 ug of immunogen in complete Freund's adjuvant. 100 ug - boosters in incomplete Freund's adjuvant were given at one week intervals for three weeks. A final 200 ug - booster was given without adjuvant 3 days before fusion. At about 8 week, spleens were removed and the mouse spleen cells were fused with P3X653-AG8.653 (mouse myeloma cell line).

P3X653-AG8.653 was maintained (before fusion) at log phase growth in a medium of 90% Dulbecco's modified Eagle's medium (high glucose) and 10% Fetal bovine serum (FBS). The following recommended supplements were added to 475 ml of the above medium: glutamine (100x) 5 ml, sodium pyruvate (100x) 5 ml, nonessential amino acids (100x) 5 ml, Pen-strep-fungizone (100x) 5 ml, and 8-azaguanine 6.6 x $10^{-3}$M (50x) 10 ml. Spleen and myeloma cells were washed throughly without FBS in Dulbecco's modified Eagle's medium before fusion. Cells were fused with 1 ml 40% PEG 1500 for 1 minute. Then cells were diluted 1:2 with growth medium for 1 minute. Cells were diluted further 1:5 with growth medium for 2 minutes. Next cells were spun at 900 RPM for 10 minutes. The supernatant was removed, the cells were selected by suspension in HAT medium and placed in 96 well plates. The HAT medium contained 90% Dulbecco's modified Eagle's medium (high glucose), 10% FBS and the following recommended supplements added to 405 ml of the above two components: glutamine (100x) 5 ml. NCTC 109 50 ml, sodium pyruvate (100x) 5 ml, nonessential amino acids (100x) 5 ml, Pen-strep-fungizone (100x) 5 ml, (hypoxanthine $10^{-2}$M + thymidine 1.6 x $10^{-3}$M) (100x) 5 ml, bovine insulin (20 I.U./ml)(100x) 5 ml, oxaloacetate ($10^{-1}$M) (100x) 5 ml. and aminopterin (2x$10^{-5}$M) (50x) 10 ml. For 4 weeks following selection the cells were maintained in growth medium - HT (selection medium minus aminopterin). Subcloning was accomplished by limiting dilution. Wells with growth were tested by ELISA assay. Test plates were coated with 100 ng/well of immunogen. 50 ul of culture supernatants were tested. Those wells containing cells whose supernatants were positive for immonogen were grown at 37°C in 10% $CO_2$.

For ascites production the mice were primed with 0.5 ml pristine at least 4 days before cell injection. The cells were injected intraperitoneally (5x$10^6$/mouse) in 0.5 ml media with no FBS. The ascites were harvested when the mice bloated. The monoclonal antibody J31B3 contained in the mouse ascites is of the IgG-1 type.

The following Protein A sepharose purification of monoclonal antibody J31B3 from mouse ascites is a modified procedure of that disclosed in Ey et al., Immunochemistry 15: 429-436 (1978). The amounts used were for a column 1 cm x 15 cm which bound about 25-30 mg IgG-1, but which allowed separation of about 50 mg IgG-1 from non-IgG proteins. The column can also bind 50 mg of IgG2a. IgG2b also binds to the column, but IgM, IgA and IgE do not bind. 4-6 ml of ascites was centrifuged at 30,000 rpm for 45 minutes. The lipids were removed on top. The addition of 20% sucrose weight/volume to the ascites aided in the removal of lipids. Ascites was diluted to 25-30 ml with 140 mM $NaPO_4$ buffer, pH = 8, containing 0.02% $NaN_3$. The ascites was diluted to prevent the interference of chloride ion with the binding of IgG. Approximately 2 g of Protein A sepharose (Sigma) was swollen in 10 mM phosphate buffered saline with 0.02% $NaN_3$ and packed into a 1 cm diameter column. The column was equilibrated in 140 mM $NaPO_4$ butter with 0.02% $NaN_3$. The column was loaded with diluted ascites at 0.06-0.08 ml/min or less. The column was allowed to sit at 4°C overnight after loading to increase binding of IgG. The column was washed with buffers at 0.6-0.8 ml/min in the following order: 1) 140 mM $NaPO_4$, pH 8.0; 2) 0.1M Na citrate - citric acid, pH 6.0 (IgG-1 eluted); 3) 0.1 M Na citrate-citric acid, pH 5.0 - IgG2a eluted and a small percentage of remaining IgG-1; 4) 0.1 M Na citrate-citric acid, pH 4.0 (small percentage of remaining IgG2a eluted); and 5) 0.1M Na citrate-citric acid, pH 3.0 (IgG2b eluted). As soon as the column was washed with pH 3.0 buffer, it was washed with 140 mM $NaPO_4$ buffer, pH 8.0 + 0.02% $NaN_3$ until the pH of the effluent was 8.0. The column was stored at 4°C. During the washing of the column approximately 5 ml fractions were collected. To any fraction of pH 5.0, 1 ml of 1M tris HCl, pH 9.0 was added.

The ability of peptides based on the amino acid sequence of Factor VIII and a sequential subset of at least three amino acids of the peptides to interfere with the binding of Factor VIII to monoclonal antibodies with the ability to bind Factor VIII can be used in the purification of Factor VIII. Thus, the ability of peptide TDSEMDVVRFDDDNS to interfere with the binding of monoclonal antibody C5 to Factor VIII was shown in an assay in which TDSEMDVVRFDDDNS blocked C5 from neutralizing Factor VIII activity.

A solution of TDSEMDVVRFDDDNS (22.5 ul) dissolved in barbital saline buffer (pH 7.56), and 22.5 ul of barbital saline was mixed with 5 ul (total 10 ug) of C5 in barbital saline and incubated overnight at room temperature. As controls, barbital saline or other peptides were substituted for TDSEMDVVRFDDDNS, other peptides were substituted for the 22.5 ul of barbital saline, or 5 ul of barbital saline was substituted for C5. After the overnight incubation, 50 ul of normal plasma containing Factor VIII was added to the TDSEMDVVRFDDDNS-C5 mixture and incubated for 2 hours at 37°C. Factor VIII activity remaining in the normal plasma was then measured in an activated partial thromboplastin time assay using an MLA electra 700 automatic coagulation timer. In that assay, the shorter the clotting time, the more Factor VIII is present in the test mixture.

In a typical experiment, incubation of barbital saline (50 ul) with plasma (50 ul) resulted in a clotting time of 66.6 and 65.6 seconds in duplicate assays. This represents the amount of Factor VIII activity remaining if no Factor VIII is inactivated by C5. If C5 and barbital saline were incubated with plasma the duplicate clotting times were 83.4 and 84.0 seconds. This represents inactivation of Factor VIII by C5. However, if C5 was first incubated with a mixture containing 22.5 ul of 1 mM TDSEMDVVRFDDDNS the clotting time was reduced to 71.1 seconds, indicating partial blocking of C5. It the concentration of TDSEMDVVRFDDDNS was increased to 6mM the clotting time was decreased to 67.1 seconds indicating almost complete neutralization of C5.

The following examples are further illustrative of the present invention. These examples are not intended to limit the scope of the present invention, but provide further understanding of the invention.

### Example 1

The peptide TDSEMDVVRFDDDNS can be used in the purification of Factor VIII. Monoclonal antibody C5 or any antibody which binds to Factor VIII, and in which binding to Factor VIII can be blocked with the peptide TDSEMDVVRFDDDNS or a subset of it, is linked to a support. Typically this is a solid phase support such as agarose, however other supports might be used effectively. A column is prepared from the antibody-support and a source of Factor VIII is passed through it. This source could be plasma, a concentrate of plasma, or Factor VIII produced by recombinant DNA techniques. The antibody-column is then washed, leaving Factor VIII bound to the antibody-column. The peptide TDSEMDVVRFDDDNS is then applied to the column. This will displace the bound Factor VIII, thus eluting the Factor VIII in a highly purified state.

### Example 2

Monoclonal antibody J31B3 raised against the peptide EEIDYDDTISVEMKK was coupled to Sepharose 4B beads by the cynaogen bromide technique.

Cyanogen bromide-activated Sepharose 4B (Sigma) was swollen in 1mM HCl for 15 minutes and then washed with 300 ml of 1mM HCl. After a brief wash with coupling buffer (0.2M sodium bicarbonate, 0.5 M sodium chloride, pH 9.0), approximately 1 ml of the resin was mixed with 5.4 mg of purified J31B3 monoclonal antibody in coupling buffer. The resin and antibody were mixed together overnight at 4°C on a rotator. Coupling was determined by comparison of the absorption of pre and post coupling supernatants at 280 nm. The coupled resin was then mixed in a similar manner for 2 hours in coupling buffer containing 0.2 M glycine to block any remaining active groups. The coupled resin was then washed 5 times alternately with coupling buffer and acetate buffer (0.1 M sodium acetate, 0.5 M sodium chloride, pH 4) to remove any noncovalently bound antibody. The coupled resin was then pre-eluted with 1 ml of 3 M sodium thiocyanate (in 0.05 M sodium acetate buffer, pH 5.5, containing 0.1 M lysine). This chaotropic solution further ensured removal of any noncovalently bound antibody. The coupled resin was then washed and stored at 4°C in imidazole buffered saline (0.02 M imidazole, 0.15 M sodium chloride, 0.02% sodium azide, pH 7.0).

A 1.1 milliliter column of J31B3-Sepharose was then constructed and washed with imidazole buffered saline. 2.5 ml of a solution containing 0.9 units/ml of Factor VIII, purified according to the technique set forth in United States Patent No. Re. 32,011, was then applied. No Factor VIII activity was found in the buffer passing through the column, indicating that all of the Factor VIII had bound to the column. The column was then washed with imidazole buffered saline and imidazole buffered saline containing 1% bovine serum albumin. The Factor VIII was then eluted with a solution of 0.25 molar calcium chloride in imidazole buffered saline containing 1% bovine serum albumin. The Factor VIII was eluted in two 500 ul fractions with a concentration of 2.24 units/ml and 0.57 units/ml, respectively. Thus Factor VIII was bound by the column, and eluted in a more concentrated state than when it went on. The recovery was 62% of the Factor VIII applied to the column.

The foregoing experiment demonstrated that a monoclonal antibody raised against the peptide EEIDYDDTISVEMKK could be bound to a solid matrix and in this state bind Factor VIII from a solution. Furthermore, Factor VIII could be eluted from the antibody by 0.25 molar calcium chloride, an agent sufficiently mild that 62% of activity was recovered.

### Example 3

The peptide EEIDYDDTISVEMKK can be used in the purification of Factor VIII. Monoclonal antibody J31B3, or any antibody raised against the peptide with the amino acid sequence EEIDYDDTISVEMKK and which binds to Factor VIII, and which binding to Factor VIII can be blocked with the peptide EEIDYDDTISVEMKK, is linked to a support. Typically this is a solid phase support such as agarose, however other supports might be used effectively. A column is prepared from the antibody-support and a source of Factor VIII is passed through it. This source could be plasma, a concentrate of plasma, or Factor VIII produced by recombinant DNA techniques. The antibody-column is then washed, leaving Factor VIII bound to the antibody-column. The peptide EEIDYDDTISVEMKK is then applied to the column. This will displace the bound Factor VIII, thus eluting it in a highly purified state.

### Claims

1. A peptide having an amino acid sequence corresponding to a portion of the sequence of Factor VIII, wherein said peptide can be used for the purification of Factor VIII.

2. A peptide according to Claim 1 which can be used to raise monoclonal or other antibodies for the purification of Factor VIII.

3. A peptide according to Claim 1 which can be used to elute Factor VIII from monoclonal or other antibodies for the purification of Factor VIII.

4. A peptide according to Claims 1, 2 or 3 wherein said peptide has the following amino acid sequence:
TDSEMDVVRFDDDNS
which amino acid sequence is that of amino acid residues 351-365 of the Factor VIII sequence.

5. A peptide according to Claims 1, 2 or 3 wherein said peptide has the following amino acid sequence:
EEIDYDDTISVEMKK
which amino acid sequence is that of amino acid residues 1660-1674 of the Factor VIII sequence.

6. A peptide comprising a sequential subset of at least three amino acid residues of a peptide according to any of Claims 1-5.

7. A method of preparing the peptide according to Claims 1-6 by recombinant DNA or synthetic peptide techniques.

8. A method of preparing Factor VIII comprising:

(a) absorbing Factor VIII from a recombinant produced, plasma or commercial concentrate source onto particles bound to a monoclonal antibody raised against a peptide as claimed in any of claims 1-6;

(b) eluting the absorbed Factor VIII; and

(c) recovering the highly purified Factor VIII.

9. The method according to Claim 8 wherein said monoclonal antibody is raised against a peptide of the following amino acid sequence:
TDSEMDVVRFDDDNS,
which amino acid sequence is that of amino acid residues 351-365 of the Factor VIII sequence, or any sequential subset of at least three amino acids of said peptide.

10. The method according to Claim 8 wherein said monoclonal antibody is raised against a peptide of the following amino acid sequence:
EEIDYDDTISVEMKK,
which amino acid sequence is that of amino acid residues 1660-1674 of the Factor VIII sequence, or any sequential subset of at least three amino acids of said peptide.

11. A method of preparing Factor VIII comprising:

(a) absorbing Factor VIII from a recombinant-produced, plasma or commercial concentrate source onto particles bound to a monoclonal antibody which binds Factor VIII;

(b) eluting the absorbed Factor VIII with a peptide as claimed in any of claims 1-6; and

(c) recovering the highly purified Factor VIII.

12. The method according to Claims 8 or 11 wherein the eluant used in step (b) is a peptide of the following amino acid sequence:
TDSEMDVVRFDDDNS,
which amino acid sequence is that of amino acid residues 351-365 of the Factor VIII sequence, or any sequential subset of at least three amino acids of said peptide.

13. The method according to Claims 8 or 11 wherein the eluant used in step (b) is a peptide of the following amino acid sequence:
EEIDYDDTISVEMKK,
which amino acid sequence is that of amino acid residues 1660-1674 of the Factor VIII sequence, or any sequential subset of at least three amino acids of said peptide.

14. The method according to Claim 8 wherein the eluant used in step (b) is a peptide as claimed in any of claims 1-6.

15. A method according to Claim 8 wherein the eluant used in step (b) is a saline solution.

16. The method according to Claim 15 wherein the saline solution is calcium chloride.

17. The method according to Claim 16 wherein the concentration of the calcium chloride solution used in step (b) ranges from about 0.25M to about 0.5M.

18. The method according to Claims 8 or 11 wherein said absorbent particles in step (a) are agarose.